# Europäisches Patentamt

## European Patent Office

## Office européen des brevets

(19)

(11) Veröffentlichungsnummer: **0 002 028**
**B1**

(12) **EUROPÄISCHE PATENTSCHRIFT**

(45) Veröffentlichungstag der Patentschrift:
**01.04.81**

(51) Int. Cl.³: **A 61 B 5/14,** A 61 M 25/00

(21) Anmeldenummer: **78101328.9**

(22) Anmeldetag: **08.11.78**

(54) **Vorrichtung zur Blutentnahme.**

(30) Priorität: **11.11.77 DE 2750454**

(43) Veröffentlichungstag der Anmeldung:
**30.05.79 Patentblatt 79/11**

(45) Bekanntmachung des Hinweises auf die Patenterteilung:
**01.04.81 Patentblatt 81/13**

(84) Benannte Vertragsstaaten:
**BE CH FR GB LU NL SE**

(56) Entgegenhaltungen:
**FR-A-1 586 087**
**FR-A-2 038 469**
**FR-A-2 235 315**
**GB-A-1 154 623**
**US-A-2 572 314**
**US-A-3 491 748**
**US-A-3 570 484**
**US-A-3 906 930**
**US-A-3 996 923**

(73) Patentinhaber: **Walter Sarstedt**
**Kunststoff-Spritzgusswerk,**
**D-5223 Nümbrecht/Rommelsdorf (DE)**

(72) Erfinder: **Bethkenhagen, Jürgen, Heideweg 15,**
**D-5223 Nümbrecht (DE)**
Erfinder: **Kolpe, Dieter, Rosenfeldstrasse 1,**
**D-5276 Wiehl 1 (DE)**

(74) Vertreter: **Dahlke, Werner, Dipl.-Ing. et al, Frankenforster**
**Strasse 137, D-5060 Bergisch Gladbach 3 (DE)**

Vorrichtung zur Blutentnahme

Die Erfindung betrifft eine Vorrichtung zur Blutentnahme mit einer in ein Gehäuse eingesetzten Einstechkanüle, einem auf deren hinteres Ende aufgeschobenen Schlauch zur Weiterleitung des Blutes in einen Entnahmebehälter und einem Klemmhebel mit Vorsprung zum wahlweisen Abquetschen bzw. Freigeben des Schlauches.

Bei derartigen Vorrichtungen sollte eine saubere und infektionsfreie Blutentnahme aus der Vene oder Arterie gewährleistet sein. Wenn während der Blutentnahme der Entnahmebehälter gewechselt werden muss, ist es im Sinne einer sauberen und infektionsfreien Blutentnahme erforderlich, zwischen die Einstechkanüle und den Entnahmebehälter ein absperrbares Ventil einzuschalten.

Eine Vorrichtung der eingangs beschriebenen Art ist durch die FR-A-15 86 087 bekannt geworden. Der dort zum Abquetschen des Schlauches vorgesehene Klemmhebel wird durch eine Feder vom Schlauch abgehoben und kann manuell entgegen der Kraft dieser Feder so verschwenkt werden, dass sein Vorsprung den Schlauch abquetscht. Diese bekannte Vorrichtung ist jedoch dann nicht zufriedenstellend, wenn die Person, die die Blutentnahme vornimmt, beim Auswechseln des Entnahmebehälters beide Hände benötigt und infolgedessen das durch den Schlauch gebildete Ventil nicht mehr durch fortdauernden Druck auf den Hebel geschlossen halten kann.

Der Erfindung liegt die Aufgabe zugrunde, die vorgenannte bekannte Vorrichtung so weiterzubilden, dass bei einem Wechsel des Entnahmebehälters automatisch ein Abquetschen des Schlauches erfolgt und die vorgenannten Schwierigkeiten nicht auftreten.

Diese Aufgabe wird erfindungsgemäss dadurch gelöst, dass der Klemmhebel federnd gegen den Schlauch angedrückt ist und durch einen in das Gehäuse einschiebbaren Entnahmebehälter vom Schlauch abhebbar ist. Der Klemmhebel ist zu diesem Zweck an seinem sich an den Vorsprung anschliessenden freien Ende, das beim Einschieben des Entnahmebehälters diesem benachbart ist, abgeknickt und so geformt und bemessen, dass beim Einschieben eines Entnahmebehälters dessen obere Kante auf den Hebel auftrifft und ihn so nach aussen verschwenkt, dass sich sein Vorsprung von dem Schlauch abhebt und dadurch den Durchfluss freigibt. Der Entnahmebehälter wird dabei von der dem freien Ende des Klemmhebels gegenüberliegenden Seitenwand des Gehäuses parallel zur Längsrichtung der Vorrichtung geführt und abgestützt.

Der Gedanke, bei einer Blutentnahmevorrichtung ein Ventil vorzusehen, das durch federnde Mittel geschlossen ist und durch das Einführen des Entnahmebehältes geöffnet wird, ist bereits durch die US-A-39 96 923 offenbart worden, wenn auch bei einer anderen, erheblich komplizierteren Ventilkonstruktion. In anderer Weise ist dieser Gedanke in der Ventilvorrichtung nach der US-A-35 70 484 verwirklicht, dort allerdings in Verbindung mit einer Injektionsspritze, auf die das dort beschriebene Ventil aufgesetzt werden soll.

Die vorgenannten beiden Druckschriften geben jedenfalls keine Anregung, eine Vorrichtung nach der FR-A-15 86 087 in dieser Richtung weiterzuentwickeln.

Wie man der vorgenannten Druckschrift entnehmen kann, kann der auf das hintere Ende der Einstechkanüle aufgeschobene Schlauch mit seinem anderen Ende in den eingeschobenen Entnahmebehälter hineinragen. Vorzugsweise wird jedoch vorgeschlagen, eine Querwand im Gehäuse vorzusehen, die im Abstand zu der die Einstechkanüle tragenden Stirnwand angeordnet ist, und in dieser Querwand eine Auslaufkanüle zu haltern, die mit der Einstechkanüle durch den besagten Schlauch verbunden ist. Gegenüber einer Blutentnahmevorrichtung mit einem lose heraushängenden Schlauch bietet diese Weiterbildung den Vorteil, dass kein Verspritzen des im Schlauchende enthaltenen Blutes zu befürchten ist.

Nach erfolgter Blutentnahme wird die Einstechkanüle aus der Vene bzw. Arterie herausgezogen und die gesamte Vorrichtung fortgeworfen. Die Ausbildung der Vorrichtung ist so einfach, dass diese als billiger Massenartikel hergestellt werden kann.

Weitere Einzelheiten und Vorteile des Gegenstandes der Erfindung ergeben sich aus der nachfolgenden Beschreibung der zugehörigen Zeichnung, in der ein bevorzugtes Ausführungsbeispiel einer erfindungsgemäss ausgebildeten Kanüle schematisch dargestellt ist. In der Zeichnung zeigen:

Fig. 1 eine Kanüle in Vorderansicht;
Fig. 2 die Kanüle nach Fig. 1 in Seitenansicht und
Fig. 3 dieselbe Kanüle in Draufsicht.

In ein Gehäuse 1 aus Kunststoff ist an einer Stirnwand 1a eine Einstechkanüle 2 eingesetzt. Etwa in der Mitte des Gehäuses 1, parallel zur Stirnwand 1a, befindet sich eine Zwischenwand 1b, in die eine Auslaufkanüle 3 eingesetzt ist. Die beiden einander zugewandten Enden der Einstechkanüle 2 und der Auslaufkanüle 3 sind durch einen flexiblen Schlauch 4 miteinander verbunden. Von der Innenseite der Stirnwand 1a ragt ein Hebel 5 ins Gehäuseinnere, der etwa in Höhe der Mitte des Schlauches 4 einen dem Schlauch zugewandten Vorsprung 5a trägt. In der entspannten oder Ruhestellung des Hebels 5 quetscht der Vorsprung 5a den Schlauch 4 ab, der von einem die Stirnwand 1a mit der Zwischenwand 1b verbindenden Steg 7 abgestützt wird.

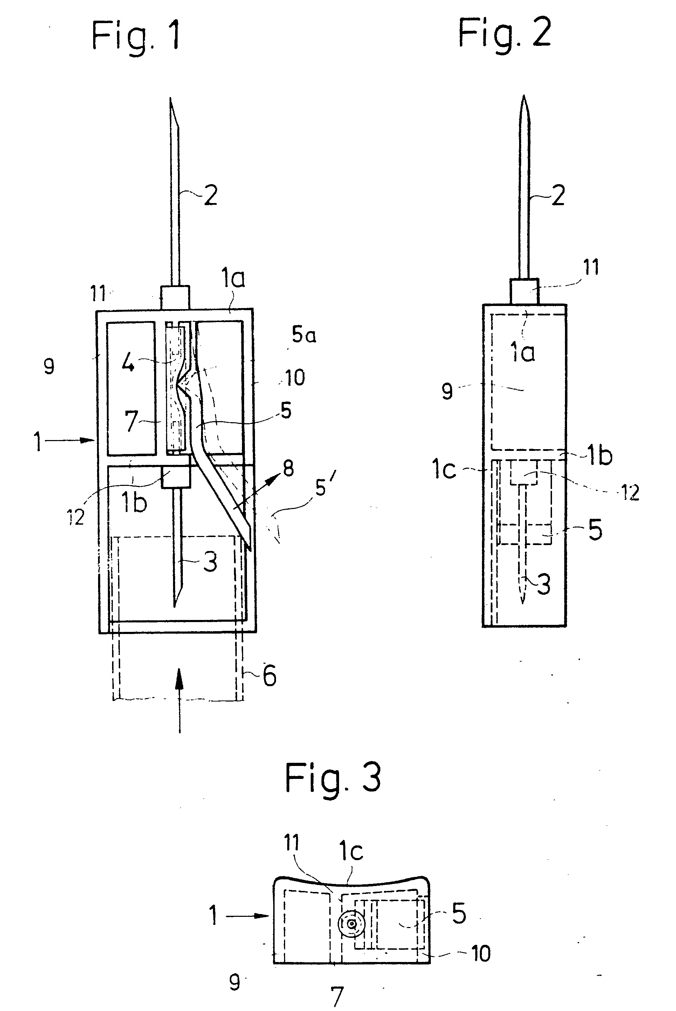

Der Hebel 5 ist, etwa in Höhe der Zwischenwand 1b, nach aussen abgebogen und erstreckt sich durch einen Durchbruch in der Zwischenwand hindurch bis zur Seitenwand des Gehäuses 1, die dort ebenfalls eine Ausnehmung besitzt. Die Anordnung ist so getroffen, dass beim Einschieben eines Röhrchens 6, das in Fig. 1 der Zeichnung gestrichelt dargestellt ist, der obere Rand dieses Röhrchens auf das freie Ende des Hebels 5 trifft und beim weiteren Einschieben den Hebel entgegen seiner Federkraft in Richtung des Pfeiles 8 nach aussen verbiegt. In Fig. 1 ist der Zeitpunkt dargestellt, in dem der obere Rand des Röhrchens 6 gerade auf den Hebel auftritt. Beim weiteren Aufwärtsschieben des Röhrchens 6 wird der Hebel in die in Fig. 1 strichpunktiert eingezeichnete Lage 5' gebracht. Dabei gibt der Vorsprung 5a am oberen Ende des Hebels die Wand des Schlauches 4 frei und der Durchfluss wird ebenfalls freigegeben.

Wenn das Röhrchen 6 nach Entnahme der gewünschten Blutmenge wieder aus dem Gehäuse 1 nach unten herausgezogen wird, federt der Hebel 5 wieder in seine ausgezogen dargestellte Lage zurück, quetscht mit seinem Vorsprung 5a den Schlauch 4 ab und schliesst damit das Ventil.

Die hintere Wand 1c des Gehäuses 1, die mit den Seitenwänden 9 und 10, der Stirnwand 1a, der Zwischenwand 1b und dem Steg 7 einstückig verbunden ist, ist, wie aus Fig. 3 zu sehen ist, leicht konkav ausgebildet. Die Vorderseite des Gehäuses ist dagegen offen ausgebildet, um die Lage des Hebels und den Durchfluss des Blutes beobachten zu können.

Zur Aufnahme der Einstechkanüle 2 steht von der Stirnwand 1a ein zylindrischer Vorsprung 11 vor, in dessen Längsbohrung die Einstechkanüle 2 fest eingesetzt ist.

In entsprechender Weise trägt die Zwischenwand 1b unterseitig einen zylindrischen Vorsprung 12, in dessen Längsbohrung die Auslaufkanüle 3 eingesetzt ist.

## Patentansprüche

1. Vorrichtung zur Blutentnahme mit einer in ein Gehäuse eingesetzten Einstechkanüle, einem auf deren hinteres Ende aufgeschobenen Schlauch zur Weiterleitung des Blutes in einen Entnahmebehälter und einem Klemmhebel mit Vorsprung zum wahlweisen Abquetschen bzw. Freigeben des Schlauches, dadurch gekennzeichnet, dass der Klemmhebel (5) federnd gegen den Schlauch (4) angedrückt ist und durch einen in das Gehäuse (1) einschiebbaren Entnahmebehälter (6) vom Schlauch abhebbar ist.

2. Vorrichtung nach Anspruch 1, gekennzeichnet durch eine Querwand (1b) im Gehäuse (1), die im Abstand zu der die Einstechkanüle (2) tragenden Stirnwand (1a) angeordnet ist, und durch eine in dieser Querwand gehalterte und mit der Einstechkanüle (2) durch den Schlauch (4) verbundene Auslaufkanüle (3).

## Patent Claims

1. Apparatus for blood collection comprising a puncturing syringe which is inserted into a housing, a tube which is slipped onto the rear end of the syringe to convey the blood into a collector and a clamping lever with a projection for the selective squeezing and releasing of the tube, characterized in that the clamping lever (5) is pressed resiliently against the tube (4) and can be detached from the tube by a collector (6) which can be inserted into the housing (1).

2. Apparatus according to Claim 1, characterized by a transverse wall (1b) in the housing (1), which wall is located some distance from the end wall (1a) supporting the puncturing syringe (2), and by a draining syringe (3) which is mounted in the said transverse wall and is connected to the puncturing syringe (2) by the tube (4).

## Revendications

1. Dispositif de prise de sang, comportant une aiguille creuse de ponction insérée dans un corps, un tuyau souple engainant l'extrémité postérieure de cette aiguille pour acheminer le sang vers un récipient de prélèvement et un levier de blocage muni d'une saillie par laquelle il est possible à volonté d'écraser le tuyau souple ou de le libérer, caractérisé en ce que le levier de blocage (5) est pressé par effet de ressort contre le tuyau souple (4) et peut être écarté du tuyau souple par un récipient de prélèvement (6) emboîtable dans le corps (1).

2. Dispositif selon la revendication 1, caractérisé par une cloison transversale (1b) dans le corps (1), située à distance de la paroi frontale (1a) qui porte l'aiguille creuse de ponction (2), et par une aiguille creuse d'écoulement (3) qui est maintenue dans cette cloison transversale et qui est raccordée à l'aiguille creuse de ponction (2) par le tuyau souple (4).

# Fig. 1

# Fig. 2

# Fig. 3